# EUROPÄISCHE PATENTSCHRIFT

(11) **EP 2 293 748 B1**
(45) Veröffentlichungstag und Bekanntmachung des Hinweises auf die Patenterteilung: **10.01.2018**
(21) Anmeldenummer: 09798866.1
(22) Anmeldetag: 02.11.2009
(51) Int. Cl.: A61F 9/007

(54) **VERFAHREN ZUR HERSTELLUNG EINER HÜLLE**
METHOD OF MANUFACTURING A SLEEVE
PROCÉDÉ DE PRODUCTION D'UN MANCHON

(30) Priorität: 09.12.2008 DE 102008060868
(43) Veröffentlichungstag der Anmeldung: 16.03.2011
(73) Patentinhaber: Geuder AG, 69126 Heidelberg (DE)
(72) Erfinder: DRAHEIM, René, 69207 Sandhausen (DE); GEUDER, Volker, 69126 Heidelberg (DE); FRAUENFELD, Dieter, 69121 Heidelberg (DE)
(74) Vertreter: Patent- und Rechtsanwälte Ullrich & Naumann
(86) Internationale Anmeldenummer: PCT/DE2009/001525
(87) Internationale Veröffentlichungsnummer: WO 2010/066222

(56) Entgegenhaltungen:
- EP-A1- 0 352 984
- WO-A1-00/01310
- WO-A1-98/19730
- DE-A1-102004 057 382
- US-A- 4 054 143
- US-A- 5 505 693
- US-A- 5 807 310
- US-A- 6 117 151
- US-B1- 6 299 591

## Beschreibung

Die Erfindung betrifft eine Hülle für ein medizinisches Instrument, insbesondere zum Zugang in den menschlichen oder tierischen Körper, beispielsweise in das Auge, vorzugsweise als Infusionsaufsatz für eine zum Zerkleinern und Entfernen von Gewebe dienende Ultraschallnadel, mit einem einen Anschlussbereich und einen Sondenbereich umfassenden Körper, der das Instrument bzw. die Nadel bis zu deren freien Ende mit geringem Abstand umgibt, so dass im Bereich zwischen dem Instrument bzw. der Nadel und dem Körper ein Fluidum förderbar ist, wobei der Körper vorzugsweise einteilig aus Gummi oder weichem Kunststoff gefertigt ist. Des Weiteren betrifft die Erfindung ein Verfahren zur Herstellung einer entsprechenden Hülle.

Eine gattungsbildende Hülle ist aus der WO 93/05716 bekannt. Im Konkreten handelt es sich dabei um einen Infusionsaufsatz für eine zum Zerkleinern und Entfernen von Gewebe dienende Ultraschallnadel, wobei die den Infusionsaufsatz bildende Hülle einen zum Aufstecken auf das Instrument dienenden Anschlussbereich und einen die Ultraschallnadel mit Abstand umgebenden Sondenbereich umfasst.

Hüllen bzw. Infusionsaufsätze der gattungsbildenden Art werden insbesondere bei Augenoperationen zum Zerstören und Entfernen des Linsenkörpers verwendet. Sie finden aber auch ihren Einsatz zum Entfernen von Ablagerungen in Arterien oder dergleichen. Entsprechend ihrem Einsatz dienen sie zum Einführen in den Körper durch künstliche Zugänge hindurch. Eine hinreichende Elastizität im Sondenbereich soll die Öffnungswunde schonen, insbesondere gegenüber einer möglicherweise überhitzten Ultraschallnadel im distalen Bereich.

Der Abstand zwischen der Hülle bzw. dem Sondenbereich und der Ultraschallnadel dient zum Durchfluss einer kühlenden Flüssigkeit, die am distalen Ende über das Instrument bzw. die als Hohlkörper ausgeführte Ultraschallnadel abgesaugt wird.

Bereits in der WO 93/05716 ist ein Nachteil gattungsbildender Hüllen erkannt worden, wonach nämlich eine solche Hülle zumindest im Sondenbereich hinreichend elastisch sein muss, damit sie sich zumindest geringfügig gegenüber dem distalen Ende des Instruments bzw. der Ultraschallnadel zurückschieben lässt, damit nämlich das als Werkzeug dienende distale Ende der Ultraschallnadel auch tatsächlich im Sinne eines abrasiven Werkzeugs arbeiten kann. Jedoch muss der Sondenbereich gleichzeitig eine hinreichende Festigkeit aufweisen, damit er beim Einführen in den Körper nicht zu weit zurückgeschoben bzw. zum proximalen Ende hin zurückgefaltet und gegenüber dem Instrument bzw. der Ultraschallnadel geknickt wird, so dass die kühlende Flüssigkeit nicht mehr in hinreichendem Umfange hindurchtreten kann. Gemäß WO 93/05716 ist das bekannte Problem dadurch gelöst, dass der Sondenbereich ausschließlich im Bereich des distalen Endes hinreichend elastisch gehalten ist, damit er dort, und nur dort, geringfügig gegenüber dem Instrument bzw. der Ultraschallnadel vom distalen Ende weg zurückschiebbar ist. Zum Anschlussbereich hin ist der Sondenbereich von einer Metallhülse umgeben, um nämlich dem Sondenbereich eine hinreichende Festigkeit bzw. Stabilität zu vermitteln. Die Vorkehrung einer solchen metallischen Hülse ist jedoch insoweit problematisch, als dadurch die Körperwunde in besonderem Maße gereizt, jedenfalls nicht geschont wird. Außerdem besteht die Gefahr, dass sich die metallische Hülse aufgrund der Ultraschallbewegung der ebenfalls metallischen Ultraschallnadel erwärmt oder gar erhitzt, wodurch ebenfalls ein negativer Effekt auf die Wunde hervorgerufen wird. Außerdem reicht die geringe Flexibilität des Sondenbereichs ausschließlich im Bereich des distalen Endes nicht aus, um das freie Ende der Ultraschallnadel zweckentsprechend freizugeben.
Der vorliegenden Erfindung liegt daher die Aufgabe zugrunde, eine gattungsbildende Hülle, insbesondere einen gattungsbildenden Infusionsaufsatz, derart auszugestalten und weiterzubilden, dass er sich zum schonenden Einsatz bei höchster Betriebssicherheit ganz besonders im Ultraschallbetrieb eignet. Außerdem soll ein Verfahren zur Herstellung einer entsprechenden Hülle angegeben werden.

In Bezug auf das erfindungsgemäße Verfahren ist die zuvor genannte Aufgabe durch die Merkmale des Patentanspruchs 1 gelöst. Erfindungsgemäß ist erkannt worden, dass sich eine elastische Hülle zum zweckentsprechenden chirurgischen bzw. augenchirurgischen Einsatz dann besonders eignet, wenn der Sondenbereich zwar elastisch ausgeführt ist, jedoch durch bestimmte Maßnahmen zumindest an der Oberfläche versteift ist. Eine geeignete Maßnahme ist beispielsweise die Vorkehrung einer möglichst dünnen Beschichtung. Auch ist es denkbar, die Versteifung durch Reaktion oder Diffusion zumindest an der Oberfläche des Sondenbereichs hervorzurufen. Die erforderliche Elastizität zum zumindest geringfügigen Zurückschieben des Sondenbereichs ergibt sich aus dem gesamten Sondenbereich, wobei die Beschichtung oder Reaktion bzw. Diffusion zumindest an der Oberfläche zu einer Versteifung führt, so dass ein Kollaps eines zu weichen Sondenbereichs wirksam vermieden ist. So lässt sich die erforderliche Funktion in Form eines Ringkanals, beispielsweise um eine Ultraschallnadel herum, aufrechterhalten, wobei eine Freigabe des distalen Endes der Ultraschallnadel aufgrund der verbleibenden Elastizität gewährleistet ist.
Im Konkreten ist der Körper der Hülle aus Silikon hergestellt. Eine spritzgusstechnische Herstellung ist zu bevorzugen. Weiter ist es denkbar, dass der Anschlussbereich und der Sondenbereich aus unterschiedlichen Kunststoffen, insbesondere aus unterschiedlich harten Kunststoffen, gefertigt ist. Eine gleichzeitige Herstellung mit integralen Bestandteilen lässt sich im sogenannten Overmouldingverfahren realisieren.
In besonders vorteilhafter Weise ist die Versteifung des Sondenbereichs derart bemessen bzw. ausgelegt, dass im Ultraschallbetrieb des Instruments bzw. der Ultraschallnadel ein Knicken oder Einschnüren des Sondenbereichs weitestgehend verhindert ist, jedoch ein zumindest geringfügiges Zurückschieben des Sondenbereichs, weg vom distalen Ende des Instruments bzw. der Nadelspitze, möglich ist. Die Funktion der Hülle ist damit auf einfache Weise optimiert.

Zur Beschichtung bzw. Diffusion oder Reaktion könnte ein Material mit Nanopartikeln verwendet werden, wobei sich die Verwendung von Nanopartikeln insbesondere zur Einlagerung in eine offene Porosität des Materials der Hülle besonders eignet.

Grundsätzlich lassen sich zur Beschichtung bzw. zur Erzeugung einer Reaktion oder Diffusion unterschiedlichste Materialien verwenden, wobei es lediglich auf das Ergebnis insoweit ankommt, als eine Versteifung des Sondenbereichs vorgenommen wird. Jedenfalls kann zur Beschichtung ein Kunststoff dienen, der zumindest an der Oberfläche eine Reaktion bzw. eine Brückenbildung oder Vernetzung hervorruft. Ebenso ist es denkbar, dass zur Beschichtung ein Metall verwendet wird, wobei das Metall aufgedampft, aufgesputtert oder sonst wie aufgebracht werden kann. Dabei reicht eine äußerst dünne Schicht bereits aus, um die erforderliche Versteifung des Sondenbereichs hervorzurufen.

Zur Reaktion und/oder Diffusion lässt sich ebenso ein Kunststoff verwenden, wie auch bereits zu der zuvor erwähnten Oberflächenbeschichtung.

In weiter vorteilhafter Weise lässt sich die Beschichtung bzw. das Aufbringen des zur Reaktion oder Diffusion dienenden Materials über ein Tauchbad oder durch Besprühen realisieren. Es könnte beispielsweise lediglich der Sondenbereich in das Bad eingetaucht oder mit dem zum Verspröden dienenden Kunststoff besprüht werden. Auch eine Art Pulverbeschichtung mit anschließender Wärmebehandlung ist denkbar.

Das erfindungsgemäße Verfahren dient insbesondere zur Herstellung einer erfindungsgemäßen Hülle, wobei es darauf ankommt, dass zumindest der Sondenbereich, ganz oder teilweise, geringfügig versteift wird. Diese Versteifung kann durch eine Versprödung des Materials erzeugt werden.

In Bezug auf das erfindungsgemäße Verfahren gelten die gleichen Ausführungen wie zu der erfindungsgemäßen Hülle. In Ergänzung zu den voranstehenden Ausführungen sei erwähnt, dass es von Vorteil ist, wenn das Material der Hülle bzw. des Körpers der Hülle vor dem Beschichten entfettet wird. Dazu kann vor dem eigentlichen Beschichten eine Anwendung in einem Ultraschallbad vorgenommen werden. Der Vorgang des Säuberns/Entfettens lässt sich dadurch begünstigen. Auch ist es denkbar, dass die Beschichtung bzw. das Tränken unter Ultraschalleinwirkung erfolgt, um nämlich einen besseren Kontakt zwischen der Oberfläche der Hülle bzw. des Sondenbereichs und dem zur Beschichtung dienenden Material hervorzurufen. Erfindungsgemäß wird die Hülle vor dem Beschichten mit einem Haftvermittler behandelt, um nämlich die Grenzflächenenergie zwischen dem Material der Hülle und dem zur Beschichtung dienenden Material zu reduzieren. Es gibt nun verschiedene Möglichkeiten die Lehre der vorliegenden Erfindung in vorteilhafter Weise auszugestalten und weiterzubilden. Dazu ist einerseits auf die dem Patentanspruch nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigt
- Fig. 1: in einer schematischen Ansicht ein Ausführungsbeispiel einer erfindungsgemäßen Hülle in Form eines Infusionsaufsatzes für eine zum Zerkleinern und Entfernen von Gewebe dienenden Ultraschallnadel, wobei in Fig. 1 lediglich die Nadelspitze erkennbar ist und
- Fig. 2: den Gegenstand aus Fig. 1 in gleicher Ansicht, jedoch geschnitten.

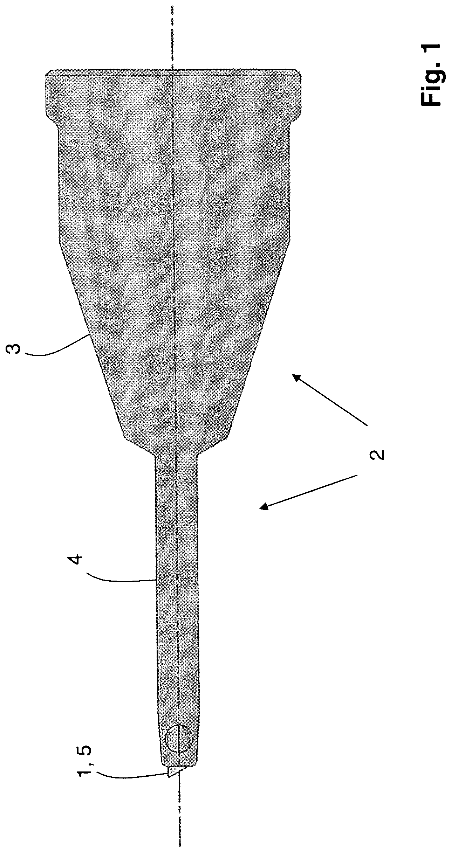

Fig. 1 zeigt ein Ausführungsbeispiel einer erfindungsgemäßen Hülle in Form eines Infusionsaufsatzes für eine Ultraschallnadel 1.
Der Infusionsaufsatzes umfasst einen Körper 2 mit einem Anschlussbereich 3 zum Aufstecken auf ein in den Fig. nicht gezeigtes Instrument und einen Sondenbereich 4, der die Ultraschallnadel 1 bis zu deren freien/distalen Ende 5 mit Abstand umgibt, so dass zwischen der Ultraschallnadel 1 und dem Körper 2 bzw. dem Sondenbereich 4 ein Ringkanal 6 realisiert ist. Durch diesen Ringkanal 6 hindurch lässt sich ein Fluidum vom proximalen Ende bzw. Anschlussbereich 3 zum distalen Ende 7 durch den Sondenbereich 4 hindurch fördern.

Fig. 1 zeigt deutlich, dass die Ultraschallnadel 1 mit ihrem distalen Ende 5 aus dem Sondenbereich 4 des Körpers 2 geringfügig herausragt, damit dort aufgrund der Ultraschallerregung ein abrasiver Effekt zum Zerstören des Gewebes auftritt. Fig. 1 zeigt des Weiteren, dass der Sondenbereich 4 am distalen Ende 7 zwei seitliche Ausgänge 8 aufweist, durch die hindurch Kühl-/Spühlmittel in die Körperöffnung hinein austreten kann. Somit lässt sich das distale Ende 5 der Ultraschallnadel 1 besonders gut umspülen, wobei die Flüssigkeit nebst abgetrenntem Gewebe durch die als Hohlnadel ausgeführte Ultraschallnadel 1 hindurch zum proximalen Ende 9 der Ultraschallnadel 1 abgesaugt wird.

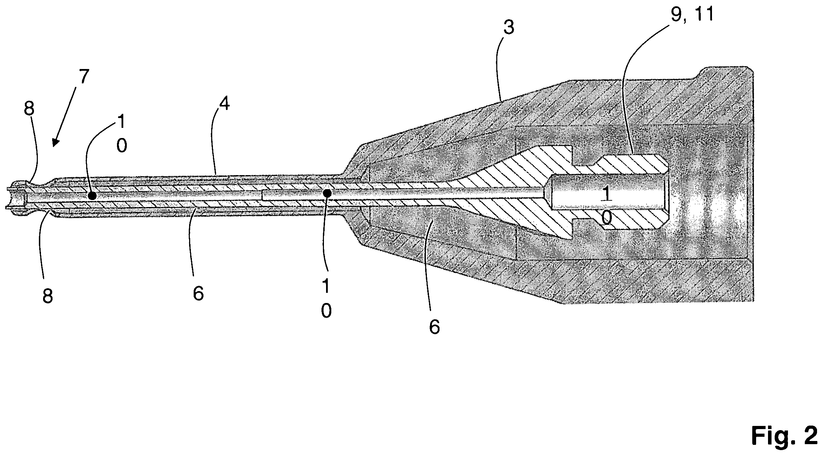

In Bezug auf die Fig. 1 und 2 sei an dieser Stelle angemerkt, dass der Anschlussbereich 3 des Körpers 2 hinreichend elastisch sein sollte, um diesen nämlich über einen entsprechenden Anschlussbereich des nicht gezeigten Instruments zu schieben. Eine Klemmwirkung ergibt sich aus der Elastizität des Materials.

Wäre der Sondenbereich 4 genauso elastisch wie der Anschlussbereich 3, bestünde die Gefahr, dass der Sondenbereich 4 auf der Ultraschallnadel 1 zu weit zurückgeschoben wird, wodurch Knicke oder Einschnürungen entstehen, die die Strömung des Kühlmittels unterbinden. Entsprechend ist der Sondenbereich 4 zumindest an der Oberfläche geringfügig versteift, nämlich durch eine besondere Maßnahme, die eine Beschichtung und/oder Reaktion und/oder Diffusion mit einem geeigneten Material umfassen kann. So ist in erfindungsgemäßer Weise ein Körper 2 angegeben, dessen Sondenbereich 4 zumindest geringfügig steifer ausgeführt ist als der Anschlussbereich 3, wenngleich beide Bereiche aus dem gleichen oder ähnlichen Material gefertigt sind.

Fig. 2 zeigt im lässt im Schnitt die Ultraschallnadel 1 erkennen, die in Längsrichtung mit einem inneren Kanal 10 durchzogen ist.

Das distale Ende 5 der Ultraschallnadel 1 ist im Sinne eines abrasiven Werkzeugs ausgestattet und dient bei gleichzeitiger Ultraschallerregung einerseits zur Zerstörung des Gewebes und andererseits zur Aufnahme und zum Absaugen der Gewebestücke.

Der Kanal 10 kann gestuft ausgeführt sein.

Am proximalen Ende 9 der Ultraschallnadel 1 ist ein Anschlussbereich 11 zum Anschließen an ein geeignetes Handgerät vorgesehen. Dieser Anschlussbereich ist bei zweckentsprechendem Einsatz durch den Anschlussbereich 3 des Körpers 2 umgeben, wodurch sich eine sichere Wirkverbindung herstellen lässt.

Hinsichtlich weiterer vorteilhafter Ausgestaltungen der erfindungsgemäßen Hülle wird zur Vermeidung von Wiederholungen auf den allgemeinen Teil der Beschreibung sowie auf die Patentansprüche verwiesen.

Schließlich sei ausdrücklich darauf hingewiesen, dass das voranstehend beschriebene Ausführungsbeispiel der erfindungsgemäßen Hülle lediglich zur Erörterung der beanspruchten Lehre dient, diese jedoch nicht auf das Ausführungsbeispiel einschränkt.

### Bezugszeichenliste

- 1: Ultraschallnadel
- 2: Körper
- 3: Anschlussbereich (des Körpers)
- 4: Sondenbereich (des Körpers)
- 5: freies/distales Ende der Ultraschallnadel
- 6: Ringkanal (zwischen Ultraschallnadel und Sondenbereich)
- 7: distales Ende (des Sondenbereichs)
- 8: seitlicher Ausgang (im Sondenbereich)
- 9: proximales Ende (der Ultraschallnadel)
- 10: Kanal (in der Ultraschallnadel)
- 11: Anschlussbereich (der Ultraschallnadel)

## Patentansprüche

1. Verfahren zur Herstellung einer Hülle für ein medizinisches Instrument, insbesondere zum Zugang in den menschlichen oder tierischen Körper, beispielsweise in das Auge, vorzugsweise als Infusionsaufsatz für eine zum Zerkleinern und Entfernen von Gewebe dienende Ultraschallnadel (1), wobei die Hülle einen Körper mit einem Anschlussbereich (3) und einem Sondenbereich (4) umfasst, der das Instrument bzw. die Nadel bis zu deren freien Ende (5) mit geringem Abstand umgibt, so dass im Bereich zwischen dem Instrument bzw. der Nadel und dem Körper (2) ein Fluidum förderbar ist, wobei der Körper (2) vorzugsweise einteilig aus Silikon gefertigt wird, **dadurch gekennzeichnet, dass** zumindest der Sondenbereich (4), ganz oder teilweise, durch Beschichtung zumindest an der Oberfläche geringfügig versteift wird, und dass die Hülle vor dem Beschichten mit einem Haftvermittler behandelt wird.

2. Verfahren nach Anspruch 1, **dadurch gekennzeichnet, dass** die Versteifung derart bemessen bzw. ausgelegt wird, dass im Ultraschallbetrieb des Instruments bzw. der Ultraschallnadel (1) ein Knicken oder Einschnüren des Sondenbereichs (4) weitestgehend verhindert ist, jedoch ein geringfügiges Zurückschieben des Sondenbereichs (4), weg vom distalen Ende des Instruments bzw. der Nadelspitze, möglich ist.

3. Verfahren nach Anspruch 1 oder 2, **dadurch gekennzeichnet, dass** zur Beschichtung ein Material mit Nanopartikeln verwendet wird,
beispielsweise aus Kunststoff oder Metall,
wobei im Falle einer metallischen Beschichtung das Metall aufgedampft oder aufgesputtert werden kann.

4. Verfahren nach einem der Ansprüche 1 bis 3, **dadurch gekennzeichnet, dass** die Hülle vor dem Beschichten entfettet und/oder
in einem Ultraschallbad behandelt wird und
dass die Beschichtung unter Ultraschalleinwirkung erfolgt.

## Claims

1. Method for producing a sleeve for a medical instrument, in particular for accessing the human or animal body, for example the eye, preferably as an infusion attachment for an ultrasonic needle (1) that is used to break up and remove tissue, the sleeve comprising a body having a connection region (3) and a probe region (4) that encloses the instrument or the needle as far as the free end (5) thereof at a small distance therefrom such that, in the region between the instrument or the needle and the body (2), a fluid can be conveyed, the body (2) preferably being produced in one piece from silicone, **characterised in that** at least the probe region (4) is slightly reinforced, completely or in part, by coating at least at the surface, and **in that** the sleeve is treated with an adhesion promoter before being coated.

2. Method according to claim 1, **characterised in that** the reinforcement is dimensioned or designed such that, in the ultrasonic mode of the instrument or the ultrasonic needle (1), kinks or constrictions in the probe region (4) are largely prevented, it being possible, however, for the probe region (4) to move slightly rearward, away from the distal end of the instrument or the needle tip.

3. Method according to either claim 1 or claim 2, **characterised in that** a material comprising nanoparticles, for example made of a plastics material or metal, is used for the coating, it being possible, in the case of a metal coating, for metal to be vapour-deposited or sputtered.

4. Method according to any one of claims 1 to 3, **characterised in that** the sleeve is degreased and/or treated in an ultrasonic bath before being coated, and **in that** the coating is carried out under the influence of ultrasound.

## Revendications

1. Procédé de fabrication d'une enveloppe pour un instrument médical, permettant en particulier d'accéder à l'intérieur du corps d'un être humain ou d'un animal, par exemple dans l'oeil, en particulier sous forme d'embout de perfusion, pour une aiguille à ultrasons (1) servant à fragmenter ou retirer du tissu, l'enveloppe comprenant un corps, avec une région de connexion (3) et une région de sonde (4), qui entoure avec un faible espacement l'instrument ou respectivement l'aiguille jusqu'à leur extrémité libre (5) de telle sorte qu'un fluide peut être transporté dans la région entre l'instrument ou respectivement l'aiguille et le corps (2), le corps (2) étant de préférence réalisé d'une seule pièce en silicone, **caractérisé en ce qu'**au moins la région de sonde (4) est, totalement ou partiellement, légèrement renforcée par revêtement au moins sur la surface et **en ce que** l'enveloppe est, avant l'opération de revêtement, traitée avec un agent d'adhérence.

2. Procédé selon la revendication 1, **caractérisé en ce que** le renforcement est dimensionné ou respectivement conçu de telle sorte que, dans le mode ultrasonore de l'instrument ou respectivement de l'aiguille à ultrasons (1), un flambage ou une striction de la région de sonde (4) est largement empêché(e) tandis qu'un léger coulissement en retour de la région de sonde (4) l'éloignant de l'extrémité distale de l'instrument ou respectivement de la pointe de l'aiguille est cependant possible.

3. Procédé selon la revendication 1 ou 2, **caractérisé en ce que**, pour le revêtement, il est utilisé un matériau à nanoparticules,
par exemple en matière plastique ou en métal,
le métal étant, dans le cas d'un revêtement métallique, déposé en phase vapeur ou pulvérisé.

4. Procédé selon l'une des revendications 1 à 3, **caractérisé en ce que**, avant l'opération de revêtement, l'enveloppe est dégraissée et/ou traitée dans un bain à ultrasons, et **en ce que** le revêtement s'effectue sous l'action d'ultrasons.
